(19) **Europäisches Patentamt**
**European Patent Office**
**Office européen des brevets**

(11) **EP 1 796 748 B1**

(12) **EUROPEAN PATENT SPECIFICATION**

(45) Date of publication and mention
of the grant of the patent:
**17.01.2018 Bulletin 2018/03**

(51) Int Cl.:
***A61L 26/00*** *(2006.01)*    ***A61L 15/28*** *(2006.01)*

(21) Application number: **05744836.7**

(86) International application number:
**PCT/GB2005/001874**

(22) Date of filing: **13.05.2005**

(87) International publication number:
**WO 2006/037938 (13.04.2006 Gazette 2006/15)**

(54) **APPARATUS FOR TREATING WOUNDS IN A BODY CAVITY**

VORRICHTUNG ZUR BEHANDLUNG VON WUNDEN IN KÖRPERHÖHLEN

APPAREIL POUR LE TRAITEMENT DE PLAIES DANS UNE CAVITE CORPORELLE

(84) Designated Contracting States:
**AT BE BG CH CY CZ DE DK EE ES FI FR GB GR HU IE IS IT LI LT LU MC NL PL PT RO SE SI SK TR**

(30) Priority: **06.10.2004 GB 0422116**

(43) Date of publication of application:
**20.06.2007 Bulletin 2007/25**

(73) Proprietor: **ArthroCare Corporation**
**Austin, TX 78735 (US)**

(72) Inventors:
 • **HUDSON, John Overton**
   **Leicester LE3 8AG (GB)**
 • **BAUER, Alberto**
   **E-29600 Marbella/Malaga (ES)**

(74) Representative: **Warren, Caroline Elisabeth et al**
**Mathys & Squire LLP**
**The Shard**
**32 London Bridge Street**
**London SE1 9SG (GB)**

(56) References cited:
 **EP-A- 0 372 966**    **EP-A1- 0 928 206**
 **EP-B1- 0 930 898**    **WO-A-00/07505**
 **WO-A-01/23653**    **WO-A-93/06855**
 **WO-A1-93/06855**    **DE-A1- 10 150 161**
 **US-A- 3 847 155**    **US-A- 4 895 559**
 **US-A- 5 630 833**    **US-A- 5 914 125**
 **US-A- 6 075 177**    **US-A1- 2002 025 921**
 **US-B1- 6 306 154**

## Description

The present invention relates to materials, methods and apparatus for treating a body cavity or passageway. In particular, the invention relates to materials, methods and apparatus for treating a body cavity which has been subjected to some sort of trauma, such as surgery, resulting in a wound surface.

There are numerous reasons why a patient's mucous membrane might have suffered trauma and benefit from such treatment. For example, the patient may have epistaxis (intra nasal bleeding). Alternatively, the patient may have undergone surgery of the nasal cavity (such as rhinoplasty, septoplasty, turbinoplasty or nasal sinus surgery, e.g. treatment of polyps in the nasal and/or sinus).

The term "nasal cavity" as used herein includes within its meaning not only the nasal cavity, including the lower, middle and upper nasal meatus and the nasal pharynx, but all other connected cavities having a mucosal membrane. Thus included within the meaning of this term is the paranasal sinuses such as the maxillary sinus (a pyramidal bilateral chamber, which is bordered inferiorly by the alveolar ridge and palate, medially by the nasal cavity, and laterally anteriorly by the cheek), the ethmoidal cell complex (bilateral air cells between the medial wall of the orbit and the lateral wall of the nose), the sphenoid sinus (a bilateral deep midline cavity), and the frontal sinus (a bilateral bony chamber, which is bordered inferiorly by the roof of the orbit, anteriorly by the skin of the forehead and posteriorly by the anterior cranial fassa).

Nasal plugging devices and, more generally, haemostatic devices for other purposes, are well known in the art for the treatment of bleeding inside body cavities and vessels (see, for example, WO 02/47558, WO 01/85037, WO0185036 and WO9857586). The aim of their use is the prevention of bleeding, that is, haemostasis, and for post operative packing to control bleeding and promote healing. Various haemostatic and packing devices are known including sponge materials which expand when wetted and balloon devices which can be expanded hydraulically or pneumatically, and can apply pressure to the source of the bleeding.

WO01/23653 describes a composite fabric comprising a combination of a gel-forming yarn and a reinforcing yarn.

EP0930898 describes viscous pasty materials suitable for use as wound-contacting layers in wound dressings and processes for their manufacture.

US6306154 relates to materials, apparatus, and methods for facilitating haemostasis within a body cavity or passageway.

Aspects of the invention are as set out in the appended claims.

Disclosed herein are materials, methods and apparatus for treating a body cavity. Although embodiments of the disclosure are particularly described with reference to the nasal cavity, it is to be understood that the various aspects of the disclosure will be of benefit to the treatment to other body cavities, particularly the ear and throat (tonsils).

Disclosed herein is an apparatus for treating a wound surface in a body cavity comprising a dispenser and characterised in that the dispenser contains a carboxymethylcellulose (CMC) gel-forming fibre or yarn.

The production of CMC and the knitting of fabrics therefrom is described in greater detail in WO 01/23653 and WO 02/47558 . CMC may be made by the chemical conversion of a variety of cellulosic materials, such as viscose rayon, cotton, etc. One cellulosic yarn suitable for the present invention is a Lyocell yarn. It is available from Spinneroff Streif AG, Zurichstrasse 170, Uathal, Switzerland. Lyocell is a solvent spun cellulose, produced from the natural cellulose in wood pulp by dissolution of the pulp in a solvent and then extruding the solution through a multiple-hole die, called a spinneret, to form a yarn comprised of a plurality of continuous strands. The solvent is vaporized in the process, leaving a continuous multi-filament yarn composed of pure cellulose.

The filaments in such a yarn may be chopped into staple form and spun into a yarn in a way similar to that used in processing cotton fibre.

In the conversion of cellulose to sodium carboxymethylcellulose, less than all of the cellulose building blocks may be converted to the sodium carboxymethylcellulose form and the degree of this conversion will dictate the degree to which a resultant CMC yarn will absorb water and form a gel therewith.

A preferred CMC is the active ingredient in "Gel Knit™" which is used in the "Rapid Rhino™" range of epistaxis and nasal dressings.

CMC gels upon contact with water, blood or body fluids, and swells to absorb such materials. CMC also facilitates blood clotting while absorbing any exudate and is, therefore, haemostatic. By "haemostatic" we mean the ability to arrest, stem or prevent bleeding by means other than induced tissue growth. Preferably, the haemostatic action includes vasoconstriction and/or blood coagulation.

In addition, it is well known that CMC is hydroscopic so it does not readily dry into clotted blood, and therefore can be removed easily without causing rebleeding. If it does dry, it can be easily re-gelled by wetting with water. The above mentioned properties of CMC are conducive to the formation of a wound-healing microatmosphere when the CMC gel is used to treat a body cavity.

Preferably the gel-forming fibres of the invention are formed into a yarn.

The word "yarn" as used herein, refers to an indefinite length of material suitable for weaving, knitting or braiding, typically comprised of one or more continuous strands of material or a multiplicity of relatively short length fibres spun into a fibre bundle of indefinite length, or some combination of continuous strands and spun fibres.

Preferably, the yarn is formed as a "soft twist" (sometimes also called "hosiery twist"), which is a yarn with less than approximately 1200 turns per metre. For the purposes of the present invention CMC yarn having from approximately 700 to approximately 900, and especially approximately 850 turns per metre, has been found to be particularly useful.

Provision of the CMC gel-forming fibres as a yarn has been found to confer unexpected benefits in terms of the ease with which the CMC can be hydrated with water or a water mixture. CMC yarn is considerably easier to hydrate to form a gel composition than CMC fibres or CMC powder. Also, a continuous length of CMC yarn is easier to hydrate than a corresponding length of CMC yarn which has been chopped into a plurality of shorter pieces. Again this is quite an unexpected finding.

Combinations of different gel-forming CMC fibres or yarns may be used within the scope of the present invention. Such combinations may be made by forming a yarn from different gel-forming fibres and/or by knitting combinations of different gel-forming yarns.

Conveniently, the gel-forming fibres of CMC may be provided as a single CMC type or as a mixture of different CMC fibre types, each type having a different degree of solubility which can range from high to insoluble.

The "solubility" of a CMC fibre or yarn as referred to herein can be determined according to the following solubility test.

A weighed amount ("starting weight") of CMC fibre or yarn is added to an excess of water under stirring. The solution so formed is then filtered to separate any solid or gelled material that has not dissolved.

Water is then evaporated off the filtrate to leave a solid residue. The solid residue is weighed and the residue weight is deducted from the starting weight to give the amount of CMC which has been dissolved. For example, if 100g CMC is the starting weight and 2g is the final residue weight, the degree of solubility will be 100 - 2 = 98%.

As referred to herein "High" solubility CMC means 95% or more; "low" solubility CMC means less than 50%, and "insoluble" CMC means less than 10% of the starting weight is lost in the solubility test described above.

By way of example, low solubility CMC gel compositions of the invention typically have a pennanency/residence time in the nasal cavity of from 8 to 15 days following treatment whereas a permanency/residence time of from 4 to 8 days is typical with a high solubility CMC gel composition.

A skilled person can control the permanency /residence time of the gel composition in the body cavity by selecting gel-forming CMC fibres or yarn having an appropriate solubility. For example, a gel composition formed from high solubility fibres or yarn will readily absorb body fluids in use and eventually lose its physical integrity and be "washed away" or dissolved from the body cavity without medical intervention. By contrast, increasing the proportion of low solubility and/or insoluble fibres or yarn will serve to increase the permanency/residence time of the gel composition in the body cavity.

The stiffness or viscosity of the gel composition can also be controlled by varying the amount of water or an aqueous mixture (solution, dispersion, etc) that is mixed with the gel-forming fibre or yarn to make the gel composition.

Preferably, the gel composition of the invention is made by mixing 1 part by weight of CMC fibre or yarn with from 4 to 12 parts by weight of water (or water mixture). For example, 1 gram of CMC yarn is mixed with from 4 to 12 grams of water. Of course, in view of the density of water a volumetric measurement is possible, with 4 to 12 grams corresponding to 4 to 12 mls. For most aqueous mixtures an approximate volumetric measurement is also suitable, as will be appreciated by a skilled reader.

The above ratio of CMC fibre or yarn to water provides a gel composition with a viscosity range which is suitable for treating body cavities.

Still further, the composition may be reinforced by the inclusion of non-gel-forming fibres.

In general, it is preferred that the CMC gel compositions of the invention have a viscosity of from $1 \times 10^2$ Pa·s up to $3.0 \times 10^3$ Pa·s ($1 \times 10^5$ centipoise up to $3.0 \times 10^6$ centipoise) as determined by the falling sphere test at 20°C and atmospheric pressure.

A convenient test method for determining whether a CMC gel composition has a viscosity suitable for use to treat a body cavity according to the invention can be described as follows:

A sample of the gel to be tested is placed into a vertical cylinder such as a glass graduated cylinder.

A steel sphere is then dropped into the gel and the time taken for it to drop a measured distance is noted.

The viscosity may be calculated as follows:

$$2\,(dp)\,\text{g}\,a^2/9v$$

Where:

$dp$ (delta p) is the difference in density between the sphere and the gel.
g is the acceleration due to gravity (numerically = 9.81)
a is the radius of the sphere.
v is the velocity of the falling sphere.

SI units for the results will be expressed in Pascal seconds (Pa s). To convert Pa s to the more commonly used centipoises (Cp) simply multiply by 1,000.
Skilled persons will appreciate that many alternative and/or modified apparatus arrangements are possible within the scope of the present invention. For example, although a syringe is preferred, the dispenser may be provided in any other suitable form, such as a flexible bag or tube.

For example, a flexible plastic bag may be provided which comprises a CMC fibre or yarn compartment and a water compartment separated by a breakable membrane. The membrane is broken to permit mixing of the CMC fibre or yarn and water to form the gel composition. Conveniently, the bag is provided with an outlet point through which the gel composition can be dispensed. The outlet port may simply be a corner or neck of the bag which is cut off to make a dispenser which resembles an icing bag.

Preferably, however the outlet port comprises a connector to permit connection to a syringe or an applicator for applying the gel composition to a body cavity.

Although termed a "dispenser" it will be appreciated that the container for the gel-forming CMC fibre or yarn of the invention is not necessarily the container from which the gel composition is dispensed to treat a body cavity. The dispenser may simply serve as a container from which the gel composition is transferred to a further container, such as a syringe, from which the gel composition is dispensed in use to treat a body cavity. Such an arrangement is described in Example 1 below.

The apparatus is preferably provided with a container and a connection means which permits fluid communication between the dispenser and container.

The dispenser and/or container is preferably a syringe and the connection means can be any means of connecting the syringe nozzles together so that water or an aqueous mixture within one syringe can be mixed with the gel-forming CMC fibre or yarn in the other to form a gel composition of the invention.

Conveniently, one or both of the syringes is graduated to permit measurement of the requisite amount of gel-forming fibre and/or water or an aqueous mixture.

The water mixture can be an aqueous solution or dispersion of a medicament.

According to a second aspect of the invention there is provided a gel composition for treating a body cavity characterised in that the gel composition is formed by mixing from approximately 4 to 12 parts by weight of water or water mixture with from approximately 0.5 to 3 parts by weight of a CMC gel-forming fibre or yarn.

More preferably, the ratio of water or water mixture to CMC fibre or yarn is from 5 to 8 parts of water or water mixture per part by weight of CMC fibre or yarn. Various optional ingredients can also be included in the final hemostatic gel composition such as preservatives and small amounts of pharmacologically active ingredients referred to herein as ("medicaments"). For example an antibiotic or antimicrobial agent such as metronidazole, silver sulphadiazine, neomycin or penicillin and antiseptic agent such as povidone iodine and anti-inflammatory agent such as hydrocortisone or triamcinolone acteonide or a skin protective agent such as a zinc oxide can be included.

Any anti-inflammatory compound may be used by the present invention. It may be a steroid or a non-steroid. Preferred anti-inflammatory compounds as described above are selected from the group consisting of cortisone (e.g. Prednisolene, Betamethasone, fluticasone, (Flutide™), fluocortine (Lenen™), flunisolid (Santaris™), Lenen™ (a water soluble powder)) cortisol (e.g. Hydrocortison Hoechst™) or biologically active derivatives of either. An example of a biologically active derivative is cortisone acetate (USP) (an ester of cortisone), which is used as an anti-inflammatory and immunosuppressant in a wide variety of disorders. Other derivatives are also included. A "derivative" in this context refers to a modified compound which retains the basic properties of the compound from which it was derived. For example anti-inflammatory activity, effects on wound healing, immunosuppression activity, levels of toxicity and stability have not been significantly changed. Also described is a component for use together with the apparatus and/or compositions of the inventions comprising a sterile container containing a sterile composition, the composition comprising an anti-inflammatory compound in the form of an aqueous solution or in a form that is dissolvable in water, at a concentration or in an amount that is effective to reduce inflammation of a mucous membrane in a patient's body cavity or suitable to be dissolved, or for dilution, to a concentration that is effective to reduce inflammation of a mucous membrane in a patient's nasal cavity.

The skilled person is able to determine an effective concentration of any given anti-inflammatory compound by routine testing.

For example, Prednisolone (a cortisone product), has a 4 times relative glucocorticoid potency, whereas Betamethasone, the most common topical cortisone for the nose, has 30 times relative glucocorticoid potency. When used in aqueous solution at a concentration suitable for delivering a 0.05 mg dose, a single dose of Betamethasone is normally given postoperatively up to 4 x 2 single doses per day, which is the equivalent of 0.4 x 30 / 4 = 3mg Prednisolone. A typical effective concentration of cortisone that may be used with the present invention may be around 10-40 mg Prednisolone per 1ml.

Alternatively, the composition of the anti-inflammatory compound can be provided in a concentrated aqueous form or powdered form, which allows the individual practitioner to dissolve/dilute the powder or solution to a concentration appropriate for the patient in hand, given the age, sex, weight and condition of the patient and the identity of the cavity being treated. For example, an aqueous solution of the anti-inflammatory compound can be provided in a form that requires dilution 2-fold, 5-fold, 10-fold, 20-fold, 50-fold, $10^2$-fold, $10^3$-fold, $10^4$-fold, $10^5$-fold, $10^6$-fold, $10^7$-fold, $10^8$-fold, or more to provide a therapeutically appropriate concentration.

The present invention also contemplates the delivery of compounds other than anti-inflammatory drugs. Other drugs considered include:- antiphogistic drugs, steroids, non-steroids, anti-histamerics, anticholinergics, antibiotics, antimycotics and botulinum toxin; the latter of which may be used as an anti-allergic drug for allergic rhinitis therapy. It will, therefore, be appreciated by the reader that reference in this application to "anti-inflammatory compound" and the like can, for the more general practice of this invention, also be read as including other compounds, such as those listed above.

Preferably the apparatus and/or gel compositions of the invention are provided in the form of a package. The package is preferably sterilised and sealed. Sterilisation may be accomplished by a variety of methods, such as exposure to ethylene oxide.

In this process the goods are placed into an airtight chamber. After spending some time to ensure that the temperature and humidity are at optimum conditions, a vacuum is drawn on the chamber. Ethylene oxide gas is introduced. This remains in the chamber for some time in order to destroy any bacteria.

The gas is then extracted and a series of vacuum and pressure cycles are given to purge all residual gas from the system.

As is explained below, the packaging is such that the gas is allowed to penetrate but bacteria are not.

Examples which embody preferred aspects of the invention will now be described with reference to the accompanying figures in which:

Figure 1 shows a syringe dispenser (1) of the invention containing CMC yarn (2). The yarn is arranged loosely within the body of the syringe and the piston (3) is in its semi-retracted position to permit uptake of water to hydrate the CMC yarn and form the CMC gel composition.

Figure 2 shows a second smaller syringe (4) container of the invention. It is graduated to permit measurement and metering of water or a water mixture into the syringe dispenser shown in Figure 1.

Figure 3 shows the syringes of Figures 1 and 2 connected by a connector (5) which permits fluid communication between the two syringes.

Figure 4 shows an applicator (6) of the invention which includes an adaptor at its proximal end for fitting to the nozzle of one of the syringes of Figure 3.

Figure 5 shows the distal end (7) of the applicator. The "tipped" profile of the distal end causes the least possible disturbance to the delicate mucous membranes which line body cavities such as the nose.

***Example** 1- **kit comprising dispenser and gel-formingfibre in dry form***

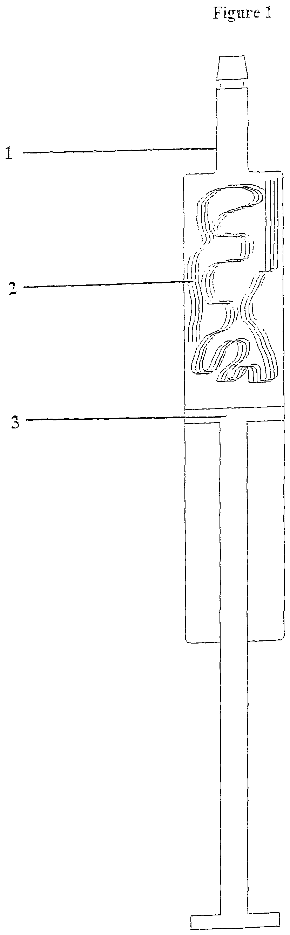

In this embodiment apparatus for treating a body cavity comprises a large (22mm diameter) syringe dispenser (1) containing a gel-forming fibre (2) consisting of 15 decitex (dtex) viscose spun yarn converted to CMC, as shown in Figure 1. The CMC yarn (1 gram) is packed loosely inside the syringe body with the piston (3) in a semi-retracted position so as not to compress the yarn inside the syringe body, whilst permitting uptake of water for mixing to form the gel composition. Packing the CMC yarn loosely in this manner facilitates easy hydration of the yarn to form a gel composition.

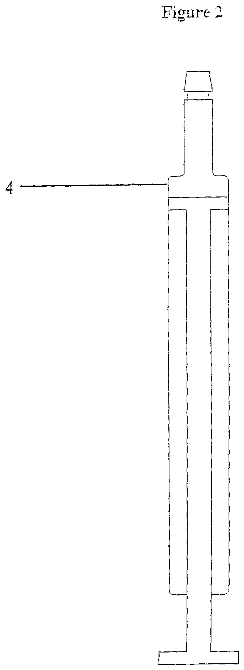

As shown in Figure 2, the apparatus further comprises a container in the form of a second smaller (10mm diameter) syringe (4) which is graduated to permit metering of a precise amount of water or water mixture (e.g. 5 to 8 mls).

The water or water mixture may be contained within the second syringe (4) or provided separately.

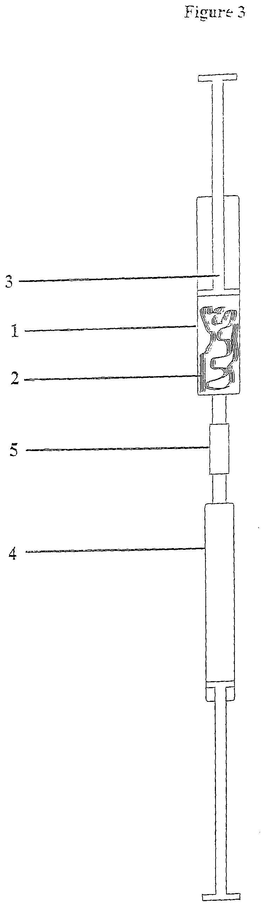

The preferred apparatus includes a connector (5), shown in Figure 3, which permits connection of the nozzle of the syringes and fluid communication therebetween, as shown in Figure 3.

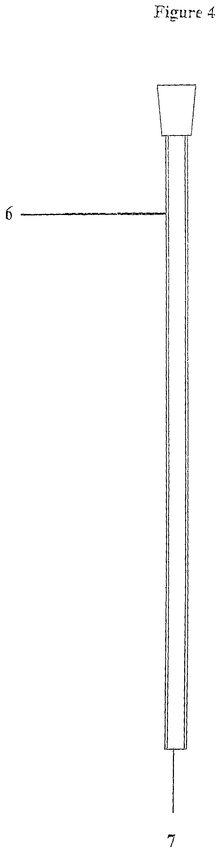

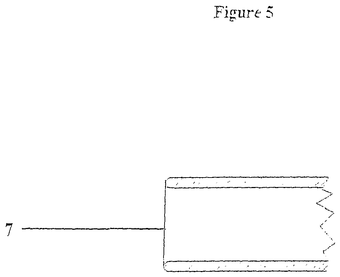

The preferred apparatus also includes an applicator (6) in the form of an elongate tubular member which is tipped at its distal end (7), as shown in Figures 4 and 5. The dimensions of the applicator are selected to permit application of a gel composition into a selected body cavity. For nasal applications the outside diameter of the applicator is preferably from 3 to 5.5mm; the internal diameter is 2 - 5mm and the length is from 80 to 140 mm.

The above apparatus is packaged and sterilised.

A typical package method is to use a thermoformed polyester blister with a tyvek™ top seal. The blister is formed so that all the individual components of the kit have their own compartment.

Tyvek™ is a proprietary semi permeable non woven polyester membrane. It is constructed such that sterilising gas is allowed through the pores in the membrane but bacteria are not allowed to pass through.

The kit may thus be packed into the seal blister/tyvek™ membrane and the contents sterilised by exposure to sterilising gas. Such a gas would typically be ethylene oxide. For sterilisation, the syringe is open, that is, any cap is left off so that the gas can come into contact with the CMC yarn.

In order to treat a body cavity the apparatus of the invention may be used as follows:

1. Measure an appropriate amount of water into the second syringe (4).
2. Connecting the two syringes together with the aid of a connector (5).
3. Holding the two syringes vertical with the water containing syringe (4) uppermost.
4. Quickly transferring the water into the fibre containing syringe (1).
5. Allowing the water to soak into the fibres - aid by gentle shaking.

6. Disconnecting the syringes and expelling excess air from the fibre containing syringe.

7. Re-connecting the two syringes with the second syringe closed.

8. Transferring the gel from syringe to syringe to mix the gel and finally collecting the gel (approximately 5 to 10 mls.) in one syringe (usually the smaller syringe).

9. Removing the empty syringe and connector.

10. Attaching the applicator (6) to the gel-filled syringe.

11. Applying the gel to the body cavity to be treated via the tipped distal end (7) of the applicator.

***Example 2 - kit comprising dispenser with pre-mixed CMC gel composition***

In this embodiment the dispenser is provided with a pre mixed CMC gel composition.

The preferred gel composition is formed as follows:

The CMC fibre and water are mixed in large quantities using standard mixing machinery common in the food and drug industries.

However, the only systems of sterilisation available to liquids or gels are those that involve radiation. (Usually gamma or X-Ray). Such radiation causes chemical degradation to CMC.

It is therefore necessary to sterilise the components of the mixture and then mix and package under sterile conditions.

After mixing, the syringes may be filled using a pressure system. The commercial process of filling syringes is well known to those in the trade.

## Claims

1. Apparatus for treating a wound surface in a body cavity comprising a dispenser (1), a container (4) and a connection means (5) which permits fluid communication between the dispenser (1) and container (4);
   wherein the dispenser contains a, CMC, carboxymethylcellulose gel-forming fibre (2); and
   wherein the container (4) is arranged to contain water or a water mixture for treating the gel-forming fibre (2) to form a haemostatic gel composition

2. Apparatus as claimed in Claim 1 wherein the dispenser (1) and/or container (4) is a syringe.

3. Apparatus as claimed in any preceding claim wherein the container (4) contains a water mixture that is an aqueous solution and/or dispersion of a medicament.

4. Apparatus as claimed in claim 3 wherein the medicament comprises an anti-inflammatory agent.

5. Apparatus as claimed in any preceding claim wherein the dispenser (1) contains a predetermined amount of gel-forming fibre (2).

6. Apparatus as claimed in Claim 5 wherein the predetermined amount is from 0.5 to 3.0 grams.

7. Apparatus as claimed in any preceding claim wherein the gel-forming fibre (2) comprises a mixture of two or more fibres having a different solubility.

8. Apparatus as claimed in any preceding claim wherein the gel-forming fibre (2) is provided together with a reinforcing fibre.

9. Apparatus as claimed in any preceding claim wherein the dispenser (1) is a syringe having a piston which is movable from a depressed position to a semi-retracted position: wherein the gel-forming fibre (2) is disposed loosely within the syringe with the piston in its semi-retracted position.

10. Apparatus as claimed in any preceding claim wherein the fibre or fibres is provided in the form of a yarn.

11. Apparatus as claimed in Claim 10 wherein the yarn is formed as a "soft twist" having at least 700 to 900 turns per metre.

12. Apparatus as claimed in any preceding claim wherein the apparatus further comprises an applicator (6) for applying gel to a body cavity.

13. Apparatus as claimed in Claim 12 wherein the applicator comprises an elongated nozzle.

14. Apparatus as claimed in Claim 13 wherein the nozzle is malleable to permit bending into a bent condition and retention of its bent condition.

15. Apparatus as claimed in any preceding claim wherein the dispenser (1) and/or container (4) is graduated to permit measurement of a defined amount of gel-forming fibre (2) and/or water and/or water mixture for treating the gel-forming fibre (2) to form a gel.

16. Apparatus as claimed in any preceding claim wherein the gel-forming fibre (2) is provided in the form of a premixed CMC gel composition.

17. Apparatus as claimed in Claim 16 wherein the gel is formed by mixing from 4 to 12 parts by weight of water or water mixture with 0.5 to 3 parts by weight of the CMC gel-forming fibre (2).

**18.** Apparatus as claimed in Claim 16 wherein the gel has a viscosity in the range from $1\times10^2$ Pa·s ($1\times10^5$ centipoise) up to $3.0\times10^3$ Pa·s ($3.0\times10^6$ centipoise) as measured by the falling sphere test when carried out at 20°C and atmospheric pressure.

**19.** Apparatus as claimed in any preceding claim wherein the apparatus is provided in the form of a sterilised package.

**20.** A sealed sterile pack comprising an apparatus according to claim 1 and an applicator (6) in the form of an elongate tubular member that is arranged to permit application of a gel composition into a body cavity.

**Patentansprüche**

**1.** Vorrichtung zur Behandlung einer Wundoberfläche in einer Körperhöhle, umfassend einen Dispenser (1), ein Behältnis (4) und ein Verbindungsmittel (5), das die Flüssigkeitskommunikation zwischen dem Dispenser (1) und dem Behältnis (4) erlaubt; wobei der Dispenser eine gelbildende Carboxymethylcellulose(CMC)-Faser (2) enthält; und wobei das Behältnis (4) derart angeordnet ist, dass es Wasser oder ein Wassergemisch zur Behandlung der gelbildenden Faser (2) zur Bildung einer hämostatischen Gelzusammensetzung enthält.

**2.** Vorrichtung nach Anspruch 1, wobei der Dispenser (1) und/oder das Behältnis (4) eine Spritze ist.

**3.** Vorrichtung nach einem der vorangehenden Ansprüche, wobei das Behältnis (4) ein Wassergemisch enthält, das eine wässrige Lösung und/oder eine Dispersion von einem Arzneimittel ist.

**4.** Vorrichtung nach Anspruch 3, wobei das Arzneimittel ein entzündungshemmendes Mittel umfasst.

**5.** Vorrichtung nach einem der vorangehenden Ansprüche, wobei der Dispenser (1) eine vorgegebene Menge von gelbildender Faser (2) enthält.

**6.** Vorrichtung nach Anspruch 5, wobei die vorgegebene Menge von 0,5 g bis 3,0 g beträgt.

**7.** Vorrichtung nach einem der vorangehenden Ansprüche, wobei die gelbildende Faser (2) ein Gemisch von zwei oder mehr Fasern mit einer unterschiedlichen Löslichkeit umfasst.

**8.** Vorrichtung nach einem der vorangehenden Ansprüche, wobei die gelbildende Faser (2) zusammen mit einer Verstärkungsfaser vorgesehen ist.

**9.** Vorrichtung nach einem der vorangehenden Ansprüche, wobei der Dispenser (1) eine Spritze mit einem Kolben ist, der aus einer hineingedrückten Position in eine halb zurückgezogene Position bewegbar ist: wobei die gelbildende Faser (2) lose in der Spritze mit dem Kolben in seiner halb zurückgezogenen Position angeordnet ist.

**10.** Vorrichtung nach einem der vorangehenden Ansprüche, wobei die Faser oder Fasern in der Form eines Garns vorgesehen ist/sind.

**11.** Vorrichtung nach Anspruch 10, wobei das Garn als eine "weiche Zwirnung" mit mindestens 700 bis 900 Drehungen pro Meter gebildet ist.

**12.** Vorrichtung nach einem der vorangehenden Ansprüche, wobei die Vorrichtung weiter einen Applikator (6) zum Auftragen des Gels auf eine Körperhöhle umfasst.

**13.** Vorrichtung nach Anspruch 12, wobei der Applikator eine längliche Düse umfasst.

**14.** Vorrichtung nach Anspruch 13, wobei die Düse verformbar ist, um das Biegen in einen gebogenen Zustand und die Beibehaltung ihres gebogenen Zustands zu erlauben.

**15.** Vorrichtung nach einem der vorangehenden Ansprüche, wobei der Dispenser (1) und/oder das Behältnis (4) graduiert ist/sind, um die Abmessung einer definierten Menge von gelbildender Faser (2) und/oder Wasser und/oder einem Wassergemisch zur Behandlung der gelbildenden Faser (2) zur Bildung eines Gels zu erlauben.

**16.** Vorrichtung nach einem der vorangehenden Ansprüche, wobei die gelbildende Faser (2) in der Form einer vorgemischten CMC-Gelzusammensetzung vorgesehen ist.

**17.** Vorrichtung nach Anspruch 16, wobei das Gel durch Mischen von 4 bis 12 Gewichtsteilen Wasser oder eines Wassergemischs mit 0,5 bis 3 Gewichtsteilen der gelbildenden CMC-Faser (2) gebildet wird.

**18.** Vorrichtung nach Anspruch 16, wobei das Gel eine Viskosität im Bereich von $1 \times 10^2$ Pa·s ($1 \times 10^5$ Cen-

tipoise) bis zu 3,0 x 10$^3$ Pa·s (3,0 x 10$^6$ Centipoise) aufweist, wie mittels des Kugelfalltests gemessen, wenn er bei 20 °C und Atmosphärendruck durchgeführt wird.

**19.** Vorrichtung nach einem der vorangehenden Ansprüche,
wobei die Vorrichtung in der Form einer sterilisierten Packung vorgesehen ist.

**20.** Verschweißte Sterilverpackung, umfassend eine Vorrichtung nach Anspruch 1 und einen Applikator (6) in der Form eines länglichen röhrenförmigen Glieds, das angeordnet ist, um das Auftragen einer Gelzusammensetzung in eine Körperhöhle zu erlauben.

**Revendications**

**1.** Appareil pour le traitement d'une surface d'une plaie dans une cavité corporelle, comprenant un distributeur (1), un contenant (4) et un moyen de raccordement (5) qui permet une communication fluidique entre le distributeur (1) et le contenant (4) ;
dans lequel le distributeur contient une fibre gélifiante de CMC, carboxyméthylcellulose (2) ; et
dans lequel le contenant (4) est agencé pour contenir de l'eau ou un mélange d'eau pour traiter la fibre gélifiante (2) afin de former une composition de gel hémostatique.

**2.** Appareil selon la revendication 1, dans lequel le distributeur (1) et/ou le contenant (4) sont/est une seringue.

**3.** Appareil selon l'une quelconque des revendications précédentes, dans lequel le contenant (4) contient un mélange d'eau qui est une solution aqueuse et/ou une dispersion d'un médicament.

**4.** Appareil selon la revendication 3, dans lequel le médicament comprend un agent anti-inflammatoire.

**5.** Appareil selon l'une quelconque des revendications précédentes, dans lequel le distributeur (1) contient une quantité prédéterminée de fibre gélifiante (2).

**6.** Appareil selon la revendication 5, dans lequel la quantité prédéterminée est de 0,5 à 3,0 gramme.

**7.** Appareil selon l'une quelconque des revendications précédentes, dans lequel la fibre gélifiante (2) comprend un mélange de deux ou plusieurs fibres ayant une différente solubilité.

**8.** Appareil selon l'une quelconque des revendications précédentes, dans lequel la fibre gélifiante (2) est fournie avec une fibre de renforcement.

**9.** Appareil selon l'une quelconque des revendications précédentes, dans lequel le distributeur (1) est une seringue comportant un piston qui peut être déplacé d'une position enfoncée à une position semi-rétractée, la fibre gélifiante (2) étant disposée de façon non serrée dans la seringue, le piston étant dans sa position semi-rétractée.

**10.** Appareil selon l'une quelconque des revendications précédentes, dans lequel la fibre ou les fibres est/sont fournie(s) sous la forme d'un fil.

**11.** Appareil selon la revendication 10, dans lequel le fil est formé comme un fil « à torsion floche » comportant au moins 700 à 900 tours par mètre.

**12.** Appareil selon l'une quelconque des revendications précédentes, l'appareil comprenant en outre un applicateur (6) pour l'application du gel dans une cavité corporelle.

**13.** Appareil selon la revendication 12, dans lequel l'applicateur comprend un embout allongé.

**14.** Appareil selon la revendication 13, dans lequel l'embout est malléable pour lui permettre de se courber afin de se retrouver dans un état courbé et de rester dans son état courbé.

**15.** Appareil selon l'une quelconque des revendications précédentes, dans lequel le distributeur (1) et/ou le contenant (4) sont/est gradué(s) pour permettre la mesure d'une quantité définie de fibre gélifiante (2) et/ou d'eau et/ou de mélange d'eau pour le traitement de la fibre gélifiante (2) afin de former un gel.

**16.** Appareil selon l'une quelconque des revendications précédentes, dans lequel la fibre gélifiante (2) est fournie sous la forme d'une composition prémélangée de gel de CMC.

**17.** Appareil selon la revendication 16, dans lequel le gel est formé par mélange de 4 à 12 parties en poids d'eau ou de mélange d'eau avec 0,5 à 3 parties en poids de la fibre gélifiante de CMC (2).

**18.** Appareil selon la revendication 16, dans lequel le gel a une viscosité de 1 x 10$^2$ Pa.s (1 x 10$^5$ centipoises) à 3,0 x 10$^3$ Pa.s (3,0 x 10$^6$ centipoises) telle que mesurée en utilisant l'essai par chute de bille réalisé à 20 °C et à la pression atmosphérique.

**19.** Appareil selon l'une quelconque des revendications précédentes, l'appareil étant fourni sous la forme d'un emballage stérilisé.

**20.** Emballage stérile hermétiquement fermé comprenant un appareil selon la revendication 1 et un applicateur (6) sous la forme d'un élément tubulaire allongé qui est agencé pour permettre l'application d'une composition de gel dans une cavité corporelle.

Figure 1

Figure 2

4

Figure 3

Figure 4

6 ——————————————————————

7

Figure 5

7 ——

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0247558 A
- WO 0185037 A
- WO 0185036 A
- WO 9857586 A
- WO 0123653 A
- EP 0930898 A
- US 6306154 B